# DEMANDE DE BREVET EUROPEEN

(11) **EP 1 374 839 A1**
(43) Date de publication de la demande: **02.01.2004**
(21) Numéro de dépôt: 03292079.5
(22) Date de dépôt: 28.08.2000
(51) Int. Cl.: A61K 7/11, A61K 7/06

(54) **Composition cosmétique comprenant au moins un copolymère silicone/acrylate et au moins un polymère non ionique à motif vinyl lactame**

(30) Priorité: 16.09.1999 FR 9911591
(62) Demande divisionnaire de: 00402376.8
(71) Demandeur: L'OREAL, 75008 Paris (FR)
(72) Inventeur: Dupuis, Christine, 75018 Paris (FR)
(74) Mandataire: Bourdeau, Françoise

(57) **Abrégé**

L'invention a pour objet une composition cosmétique, comprenant au moins un copolymère silicone/acrylate particulier et au moins un polymère non ionique comprenant au moins un motif vinyl lactame. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

## Description

L'invention a pour objet une composition cosmétique, comprenant au moins un copolymère silicone/acrylate particulier et au moins un polymère non ionique comprenant au moins un motif vinyl lactame. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

Parmi les produits capillaires, notamment ceux destinés à la mise en forme et/ou au maintien de la coiffure les plus répandus sur le marché de la cosmétique, on distingue les compositions à pulvériser essentiellement constituées d'une solution le plus souvent alcoolique ou aqueuse et d'un ou plusieurs matériaux.

Lorsque les compositions sont destinées à la fixation et/ou au maintien de la coiffure, ces matériaux sont généralement des résines polymères, dont la fonction est de former des soudures entre les cheveux, appelés encore matériaux fixants, en mélange avec divers adjuvants cosmétiques. Ces compositions sont généralement conditionnées soit dans un récipient aérosol approprié mis sous pression à l'aide d'un propulseur, soit dans un flacon pompe.

On connaît également les gels ou les mousses capillaires qui sont généralement appliqués sur les cheveux mouillés avant de faire un brushing ou une mise en plis. Pour mettre en forme et fixer la coiffure, on effectue ensuite un brushing ou un séchage. Ces gels ou mousses peuvent également contenir des résines polymères.

Toutefois, ces compositions capillaires présentent souvent l'inconvénient d'altérer les propriétés cosmétiques des cheveux. Ainsi, les cheveux peuvent devenir rêches, difficiles à démêler, perdre leur toucher et leur aspect agréables ou encore manquer de corps. On recherche donc des compositions coiffantes procurant de bonnes propriétés cosmétiques, notamment en terme de démêlage, de douceur et de toucher.

En outre, ces compositions capillaires présentent également l'inconvénient majeur de donner lieu à une effet de poudrage. Au sens de la présente invention, on entend par « poudrage », l'aptitude du matériau obtenu par séchage de la composition capillaire à former une poudre après son application sur les cheveux. Bien entendu, la poudre tombe sur les épaules ou les vêtements de l'utilisateur, ou encore elle accroche au peigne ou à la brosse, et ceci est préjudiciable.

Il existe donc un besoin de trouver des compositions cosmétiques, notamment pour le coiffage, qui ne présentent pas l'ensemble des inconvénients indiqués ci-dessus, et qui en particulier fixent bien la coiffure, tout en procurant de bonnes propriétés cosmétiques, ceci sans effet de poudrage.

De manière surprenante et inattendue, la Demanderesse a découvert que lorsque l'on associe des copolymères silicone/acrylate particulier avec certains polymères particuliers, il est possible d'obtenir des compositions cosmétiques répondant aux exigences exprimées ci-dessus.

L'invention a pour objet une composition cosmétique comprenant, dans un milieu cosmétiquement acceptable, au moins un copolymère silicone/acrylate et au moins un polymère non ionique comprenant au moins un motif vinyl lactame, le copolymère silicone/acrylate étant obtenu par polymérisation radicalaire d'au moins un monomère éthyléniquement insaturé (a) en présence d'au moins un dérivé siliconé (b) comprenant des groupements oxyalkylénés.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition pour la fabrication d'une formulation cosmétique capillaire, destinée notamment au maintien et/ou à la mise en forme de la coiffure.

Les copolymères silicone/acrylate particulièrement visés par la présente invention sont ceux décrits dans la demande internationale WO99/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule (Iₐ): 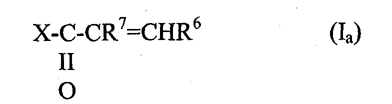 dans laquelle :
- X est choisi dans le groupe comprenant OH, OM, OR⁸, NH₂, NHR⁸, N(R⁸)₂;
- M est un cation choisi parmi Na⁺, K⁺, Mg⁺⁺, NH ⁴⁺, alkylammonium, dialkylammonium, trialkylammonium, et tétraalkylammonium;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

En particulier, les monomères (a) de formule (Ia) sont l'acide acrylique et ses sels, esters ou amides. Les esters peuvent être dérivés d'alkyles linéaires en C₁ à C₄₀, d'alkyles ramifiés en C₃ à C₄₀ ou d'alcools carboxyliques en C₃ à C₄₀, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (Ia) peuvent également être choisis parmi les N,N-dialkylaminoalkyl acrylates et méthacrylates et N-dialkylaminoalkylacryl- et -méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en C₁ à C₄₀ ou ramifiées en C₃ à C₄₀.

Les monomères (a) de formule (Ia) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en C₁ à C₄₀, les acides carboxyliques, linéaires en C3 à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique; le méthyl, éthyl, propyl, n-butyl, isobutyl, t-butyl, 2-éthylhexyl et décyl acrylate; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate; le 2,3-dihydroxypropyl acrylate; le 2,3-dihydroxypropyl méthacrylate ; le 2-dihydroxyéthyl acrylate; l'hydroxypropyl acrylate; le 2-hydroxyéthyl méthacrylate ; le 2-hydroxyéthyl éthacrylate; le 2-méthoxyéthyl acrylate; le 2-éthoxyéthyl méthacrylate ; le 2-éthoxyéthyl éthacrylate; l'hydroxypropyl méthacrylate; le glycéryl monoacrylate; le glycéryl monométhacrylate; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les bases oraganiques telles que les aminoalcools, comme les alcanolamines telle que la méthanolamine, le 2-amino-2-méthyl-1-propanol, la triéthanolamine, les diamines comme la lysine.

Les monomères (a) peuvent être aussi quaternisés lorsqu'ils possèdent un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning.

Parmi les monomères (b), on préfère ceux qui présentent la formule I ci-après : 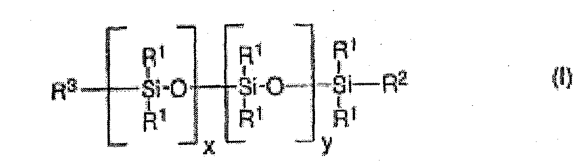 dans laquelle formule (I):
- R² est choisi parmi CH₃ ou le groupement 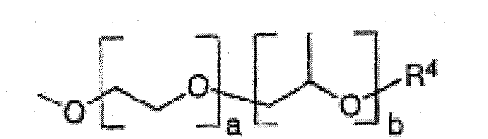
- R³ est choisi parmi CH₃ ou le groupement R²,
- R⁴ est choisi parmi l'hydrogène, CH₃, ou les groupements 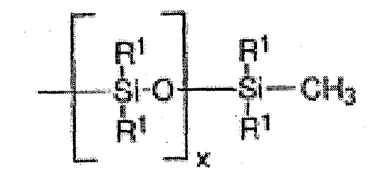 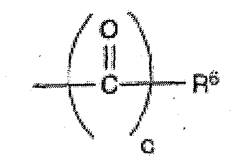
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C_{20,} et les groupements R⁵ répondant à la formule ci-après :

Parmi les dérivés siliconés (b), on préfère ceux qui présentent la formule I ci-après : 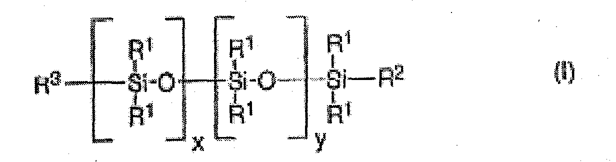 dans laquelle formule (I):
- R² est choisi parmi CH₃ ou le groupement 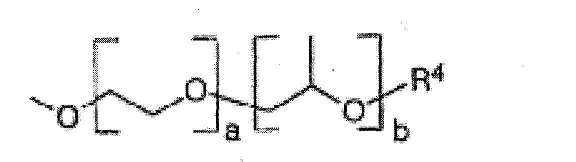
- R³ est choisi parmi CH₃ ou le groupement R²,
- R⁴ est choisi parmi l'hydrogène, CH₃, ou les groupements 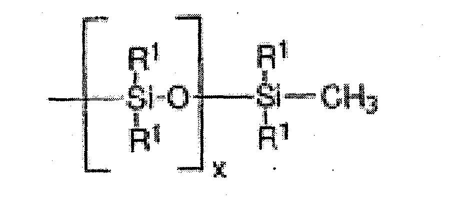 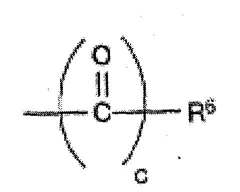
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 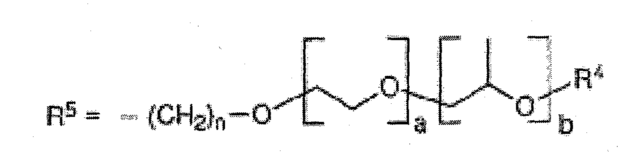
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

De préférence, R¹ est choisi parmi les groupements méthyl, éthyl, propyl, butyl, isobutyl, pentyl, isopentyl, hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux R⁴ ayant pour formule -(CO)c-R⁶, R⁶ étant un alkyl, cycloalkyl or aryl ayant 1 à 40 atomes de carbone qui peut contenir des groupements supplémentaires tels que NH₂, COOH et/ou SO₃H.

On préfère les radicaux R⁶ pour lesquels c=0 qui sont des phosphates ou des sulfates.

Les dérivés siliconés (b) particulièrement préférés sont ceux présentant la formule générale suivante :

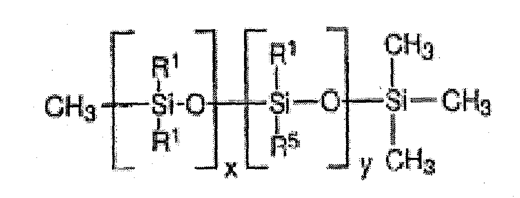

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l.

La composition comprend avantageusement, en pourcentage relatif par rapport au poids de la composition, de 0,1 à 20 % de copolymère silicone/acrylate, et plus préférentiellement de 0,5 à 10 % de ce copolymère.

Selon l'invention, les polymères à motifs vinyl lactames sont ceux qui comprennent des motifs de formule 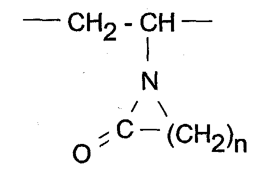 dans laquelle n est indépendamment 3,4 ou 5.

De manière avantageuse, les polymères à motifs vinyl lactames sont des copolymères ou peuvent également comprendre des motifs de formule 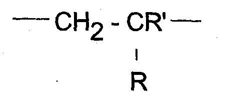 dans laquelle
R représente indépendamment un radical acyloxy, le groupement alkoxy du radical carbalcoxy pouvant être substitué par au moins un radical hydroxy ou un radical aryle, en particulier phényle, éventuellement substitué par au moins un radical alkyle, et
R' représente un atome d'hydrogène ou un radical alkyle.

Par alkyle, on entend de préférence selon l'invention les radicaux allyles linéaires ou ramifiés en C₁-C₁₀, plus préférentiellement les radicaux alkyles en C₁-C₄, en particulier les radicaux méthyle, éthyle, n-propyle, i-propyle, n-butyle et t-butyle.

Par acyle, on entend de préférence selon l'invention les radicaux acyles dont le reste alkyle est un alkyle linéaire ou ramifié en C₁-C₁₀, en particulier les radicaux acétyle ou propionyle.

Les polymères à motifs vinyl lactames selon l'invention sont notamment décrits dans les brevets US 3 770 683, US 3 929 735, US 4 521 504, US 5 158 762, US 5 506 315 et dans les demandes de brevet WO 94/121148, WO 96/06592 et WO 96/10593. Ils peuvent se présenter sous forme pulvérulente ou sous forme de solution ou de suspension.

Parmi les polymères à motifs vinyl lactames utiles selon l'invention, on citera plus particulièrement les polyvinyl pyrrolidones, les polyvinyl caprolactames, les copolymères polyvinyl pyrrolidone / acétate de vinyle, les terpolymères polyvinyl pyrrolidone / acétate de vinyle / propionate de vinyle (en particulier commercialisés sous la dénomination Luviskol VAP 343 par la société BASF.

La masse moléculaire en nombre du polymère à motifs vinyl lactames est généralement supérieure à environ 5 000, de préférence comprise entre 10 000 et 1 000 000 environ, plus préférentiellement comprise entre 10 000 et 100 000 environ.

Le polymère à motifs vinyl lactame peut être utilisés sous forme solubilisée ou sous forme de dispersions de particules solides de polymère.

La composition comprend de préférence entre 0,1 et 10 % de polymère non ionique à motif vinyl lactame, et encore plus préférentiellement entre 0,2 et 5 %, en pourcentage relatif en poids.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les tensioactifs anioniques, cationiques, non ioniques ou amphotères, les parfums, les filtres, les conservateurs, les protéines, les vitamines, les polymères autres que ceux de l'invention, les huiles végétales, minérales ou synthétiques et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'inventions peuvent être appliquées sur la peau, les ongles, les lèvres, les cheveux, les sourcils et les cils.

Les compositions conformes à l'invention sont particulièrement adaptés pour des cheveux secs ou humides, en tant que produits de coiffage.

L'invention va être plus complètement illustrée à l'aide des exemples non limitatifs suivants.

Tous les pourcentages sont des pourcentages relatifs en poids par rapport au poids total de la composition et m.a. signifie matière active.

### EXEMPLE :

On réalise la composition suivante en flacon-pompe.

| | |
|---|---|
| Luviflex Silk (BASF)* | 3 % M.A. |
| Luviskol plus** | 2% M.A. |
| AMP | qs pH= 7 |
| Ethanol | qsp 100g |

| | |
|---|---|
| * copolymère silicone/acrylate (t-butylacrylate/acide méthacrylique/PDMS polyéther) | |
| ** polyvinylcaprolactame | |

On applique cette composition par pulvérisation sur une mèche eurochâtain. On obtient un bon maintien et une très bonne cosmétique.

## Revendications

1. Composition cosmétique comprenant, dans un milieu cosmétiquement acceptable, au moins un copolymère silicone/acrylate et au moins un polymère non ionique comprenant au moins un motif vinyl lactame, le copolymère silicone/acrylate étant obtenu par polymérisation radicalaire d'au moins un monomère éthyléniquement insaturé (a) en présence d'au moins un dérivé siliconé (b) comprenant des groupements oxyalkylénés, les polymères à motifs vinyl lactames étant choisis parmi les polyvinyl pyrrolidones.

2. Composition selon la revendication 1, **caractérisée par le fait que** le monomère (a) répond à la formule (Iₐ) : 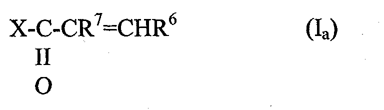 dans laquelle :
- X est choisi dans le groupe comprenant OH, OM, OR⁸, NH₂, NHR⁸, N(R⁸)₂;
- M est un cation choisi parmi Na⁺, K⁺, Mg⁺⁺, NH 4⁺, alkylammonium, dialkylammonium, trialkylammonium, et tétraalkylammonium;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

3. Composition cosmétique selon la revendication 1, **caractérisée par le fait que** le dérivé siliconé (b) répond à la formule I ci-après : 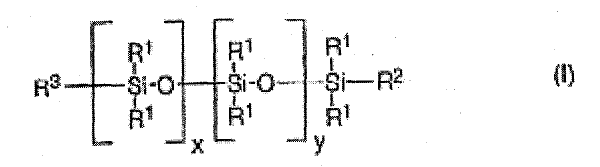 dans laquelle formule (I):
- R² est choisi parmi CH₃ ou le groupement 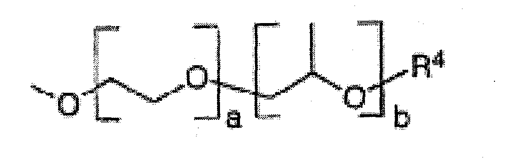
- R³ est choisi parmi CH₃ ou le groupement R²,
- R⁴ est choisi parmi l'hydrogène, CH₃, ou les groupements 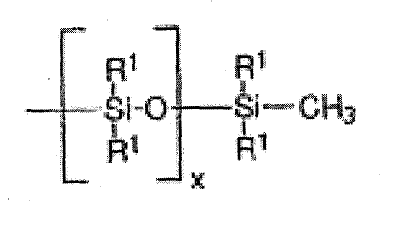 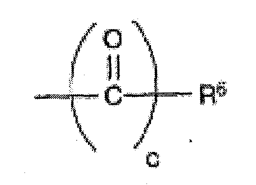
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 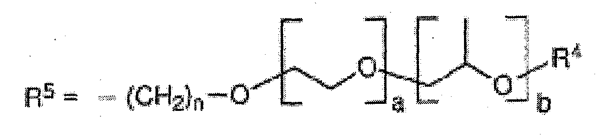
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

4. Composition selon la revendication 1, **caractérisée par le fait que** le monomère (a) est choisi parmi les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

5. Composition selon la revendication 1, **caractérisée par le fait que** le monomère (a) est choisi dans le groupe comprenant l'acide acrylique, méthacrylique et éthylacrylique; le méthyl, éthyl, propyl, n-butyl, isobutyl, t-butyl, 2-éthylhexyl et décyl acrylate; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate; le 2,3-dihydroxypropyl acrylate; le 2,3-dihydroxypropyl méthacrylate ; le 2-dihydroxyéthyl acrylate ; l'hydroxypropyl acrylate; le 2-hydroxyéthyl méthacrylate ; le 2-hydroxyéthyl éthacrylate; le 2-méthoxyéthyl acrylate; le 2-éthoxyéthyl méthacrylate; le 2-éthoxyéthyl éthacrylate; l'hydroxypropyl méthacrylate ; le glycéryl monoacrylate; le glycéryl monométhacrylate; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Composition selon l'une quelconque des revendications précédentes, **caractérisée par le fait que** les dérivés siliconés (b) sont choisis parmi les diméthicone copolyols ou les tensioactifs siliconés.

7. Composition selon l'une quelconque des revendications précédentes, **caractérisée par le fait qu'**elle comprend, en pourcentage relatif en poids, de 0,1 à 20 % de copolymère silicone/acrylate, et plus préférentiellement de 0,5 à 10 % de ce copolymère.

8. Composition selon l'une quelconque des revendications précédentes, **caractérisée par le fait que** le polymère à motifs vinyl lactames comprend des motifs de formule: 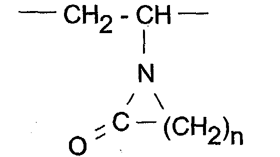 dans laquelle n est indépendamment 3,4 ou 5.

9. Composition selon l'une des revendications 1 à 7, **caractérisée en ce que** les polymères à motifs vinyl lactames sont des copolymères comprenant également des motifs de formule 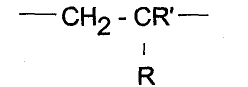 dans laquelle
R représente indépendamment un radical acyloxy, le groupement alkoxy du radical carbalcoxy pouvant être substitué par au moins un radical hydroxy, ou un radical aryle, en particulier phényle, éventuellement substitué par au moins un radical alkyle, et
R' représente indépendamment un atome d'hydrogène ou un radical alkyle.

10. Composition selon l'une quelconque des revendications précédentes, **caractérisée par le fait que** le polymère non ionique à motifs vinyl lactames est présent dans la composition à une concentration relative en poids comprise entre 0,1 et 10 %, et de préférence entre 0,2 et 5 %.

11. Composition selon l'une quelconque des revendications précédentes, **caractérisée par le fait qu'**elle contient en outre au moins un additif choisi parmi les tensioactifs anioniques, cationiques, non ioniques ou amphotères, les parfums, les conservateurs, les protéines, les vitamines, les filtres, les polymères autres que ceux de l'invention, les huiles minérales, végétales ou synthétiques.

12. Procédé de maintien ou de mise en forme de la coiffure, **caractérisé par le fait qu'**il comprend la mise en oeuvre d'une composition conforme à l'une quelconque des revendications 1 à 11.

13. Utilisation d'une composition selon l'une quelconque des revendications 1 à 11, pour la fabrication d'un produit cosmétique, en particulier capillaire.

14. Utilisation selon l'une quelconque des revendications 1 à 11 pour les cheveux, les sourcils et les cils.
